# EUROPEAN PATENT APPLICATION

(11) **EP 2 514 358 A1**
(43) Date of publication of application: **24.10.2012**
(21) Application number: 12164643.4
(22) Date of filing: 18.04.2012
(51) Int. Cl.: A61B 5/00

(54) **Medical apparatus**

(30) Priority: 20.04.2011 IT VI20110101
(71) Applicant: Studio Tecnico Per. Ind. Gianfranco Bigaran, 38062 Arco (TN) (IT)
(72) Inventor: Bigaran, Gianfranco, 38062 Arco (TN) (IT)
(74) Representative: Trentin, Michele

(57) **Abstract**

A medical apparatus, susceptible to be used on a human body to perform one or more therapeutic treatment or to perform one or more diagnostic tests, including: a programmable central control unit (2); a plurality of remote devices (4) operatively connected with the central unit (2); a plurality of interchangeable terminals (3) in combination or alternatively removably connected to the remote devices (4) and susceptible to exchange electrical, optical, acoustic and/or magnetic signals with the human body, each terminal (3) being designed to perform a specific therapeutic treatment or a specific diagnostic test different from the therapeutic treatments or the diagnostic tests executable by the other interchangeable terminals (3), each remote device (4) comprising a logical control unit (5) to interface control signals and data communications between the central unit (2) and each interchangeable terminal (3) temporarily connected to the remote device (4) to perform a specific therapeutic treatment or a diagnostic test.

## Description

### Field of application

The present invention generally relates to the technical field of the medical treatment and diagnostic devices and particularly relates to a medical apparatus.

More in detail, the present invention concerns an apparatus for the execution of particular therapies such as, for example, the Chromopuncture or laser puncture. The same apparatus can also be used to perform diagnostic tests such as electrocardiograms or the like.

### Background of the invention

Diagnostic devices and medical treatment devices are generally used in the field of the medical care of the human body. The formers are designed to valuate the physical situation of a patient to determine the state of health, whereas the latter are used to treat the patient in certain cases emerged through the tests performed with the first.

Typical examples of diagnostic devices are the devices to perform electrocardiograms, ultrasound treatment, magnetic resonance imaging devices, blood analysis devices, etc.

Typical examples of medical treatment are the devices for laser therapy, radiation therapy or others. In addition, they are diffusing alternative health treatment or complementary health treatment to traditional medicine.

Many of said methods can be attributed to the areas of analgesia or symptomatic treatment, ie in areas that take care about therapies for overcoming pain, muscle contractive or other diseases.

In all said situations, the traditional medicine offers the use of medicines, that can be NSAIDs (nonsteroidal anti-inflammatory) or No-NSAIDs (opiates, steroids) and that are intended to anesthetize different-extension parts in the human body or to cause relaxation. However, it is also known that the use of medicines involves drawbacks so that it is good practice not to abuse.

For this reason are known a number of alternative methods of treatment some common examples of which are acupuncture, some kinds of massages, cromopuncture and the like.

To facilitate the execution of such treatments, they have been developed a plurality of devices ranging from those for the laser-puncture to those for the chromopuncture or to those for the electrical stimulation.

Clearly, therefore, a clinic or a hospital authority that wishes to perform diagnostic tests and body treatments such as those mentioned above must take a plurality of devices facing high costs. In fact, They not only need of many different kinds of devices, but they also generally need to purchase multiple devices each kind to allow to make simultaneous tests or treatments.

It is also known that the current devices typically include a central unit to which are connected terminals applied to the body of the patient.

Often the central units are bulky, taking up space and proving difficult to move and place. The workplace of said diagnostic devices or said medical treatment devices includes also the patient positioning near the devices to allow the application of the terminals. Since for each patient test or therapy there must be a special closed compartment to separate it from the other patients under treatment in the same instants, it is evident that this compartment must be particularly extended to accommodate also the medical device in use. It follows that it need to provide large spaces for outpatients' clinics and hospitals, or, as generally happens, a reduction of the number of patients treatable simultaneously. It is obvious that it causes the lengthening of the waiting times.

Another drawback of the known medical devices relate to the terminals. In fact, they are generally connected to the central unit by cable. Therefore, their arrangement on the patient is uncomfortable and generally unstable.

Moreover, since generally the precision with which the terminal are arranged on the patient heavily affect on the success of the treatment or diagnostic test, it follows that for every medical device used must be present a technician or a doctor able to proceed correctly, increasing in costs.

Also in this case, however, the arrangement of the terminals can be inaccurate so as to decrease the accuracy of the test or the therapeutic treatment.

### Summary of the invention

Object of the invention is to at least partially overcome the above drawbacks by providing a medical apparatus that allows to perform simultaneously a plurality of therapeutic treatments and diagnostic tests.

Another object of the present invention is to provide a medical apparatus that will decrease the overall costs that a clinic or a hospital authority must face in order to execute said tests said therapeutic treatments.

Within said general objects, a particular object is to reduce the costs for the purchase of the devices.

Another particular object is to optimize the space required for outpatients' treatments and tests, so reducing the structural costs.

A further particular object is to decrease the number of dedicated technical or medical specialists by providing a medical apparatus whose terminals are also applicable by non-experts people.

These objects, and others which will appear more clearly below, are fulfilled by a medical apparatus in accordance with the claims that follow which are an integral part of this description.

The medical apparatus of the invention is applicable to the human body for the execution of one or more therapeutic treatment or for the execution of one or more diagnostic tests.

For this reason, it may include at least one programmable central control unit operatively connected to one or more remote devices.

To the latter may be removably coupled two or more interchangeable terminals susceptible to exchange electrical, optical, acoustic and magnetic signals with the human body. In addition, each of said terminals may be designed for the execution of a specific therapeutic treatment or diagnostic test different from those executable by other interchangeable terminals.

According to another aspect of the invention, each of the remote devices may include at least one logical control unit for the interchangeable terminals to interface control signals and data communication between the central unit and any one of the interchangeable terminals temporarily coupled to the remote device for the execution of a specific therapeutic treatment or diagnostic test.

In other words, various diagnostic tests and therapeutic treatment may be performed by a single apparatus equipped with a central unit in which reside all the electronic and information technology for the implementation and the control of these treatments and tests. This central unit may be operatively connected to remote devices which will be connected to the terminals suitable for the performance of the specific treatment or the specific diagnostic test. Advantageously, the terminals are interchangeable so that the remote devices can be always the same, and the execution of the specific test or the specific treatment can be simply achieved by coupling each time the right terminal and selecting, in the central unit, the correct settings.

Still advantageously, to allow the use of different terminals with the same remote devices, the latter include a logical control unit able to "communicate" with each different terminal and acting accordingly to the commands coming from the central unit.

To guarantee that the tests and treatments are carried out properly, between the remote devices and the central unit may be exchanged dedicated control signals to indicate the interchangeable terminal kind connected to the remote device and its operational status.

### Brief description of the drawings

Further features and advantages of the invention will appear more evident upon reading the detailed description of some preferred, non-exclusive embodiments of a medical apparatus according to the invention, which are described as non-limiting examples with the help of the annexed drawings, in which:
FIG. 1 represents a medical apparatus according to the invention in axonometric view;
FIGS. from 2 to 4 represent details of the medical apparatus of FIG. 1;
FIG. 5 represents an operating moment of the medical apparatus of FIG. 1.

### Detailed description of some preferred embodiments of the invention

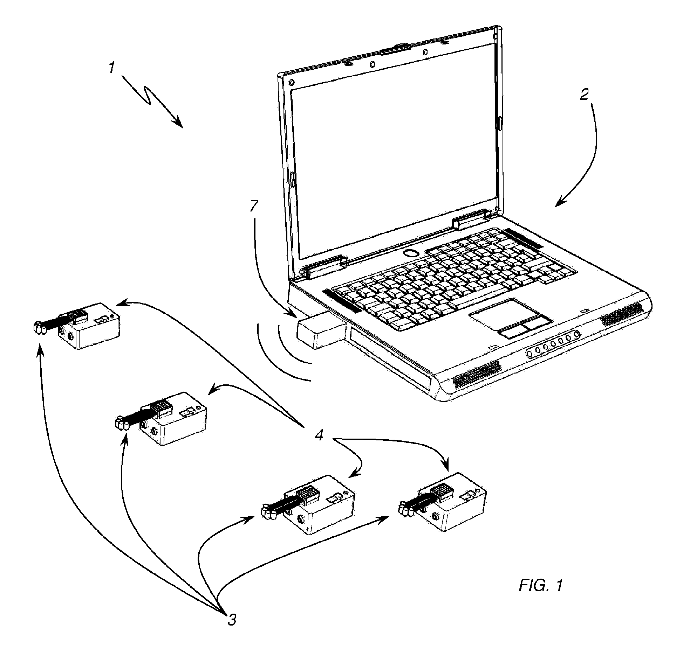

Referring to the above figures, and particularly to FIG. 1, a medical apparatus 1 it is described a medical apparatus 1 which is usable on the human body for the execution of one or more therapeutic treatment or for the execution of one or more diagnostic tests.

As mentioned above, some examples of therapeutic treatment consist on chromopuncture, electroacupuncture, laser puncture, electrostimulation and the like.

An example of a diagnostic test is the electrocardiogram.

In order to ensure that the medical apparatus **1** of the invention may perform a plurality of tests or treatments such as those just mentioned, it includes a programmable central control unit **2.** In this sense, the software running on it allows the selection and parameterization of tests and treatments.

According to one aspect of the invention, the medical apparatus **1** comprises a plurality of interchangeable terminals **3** susceptible to exchange electrical optical, acoustic and/or magnetic signals with the human body. Particularly, each terminal **3** is designed to execute a specific therapeutic treatment or a specific diagnostic test.

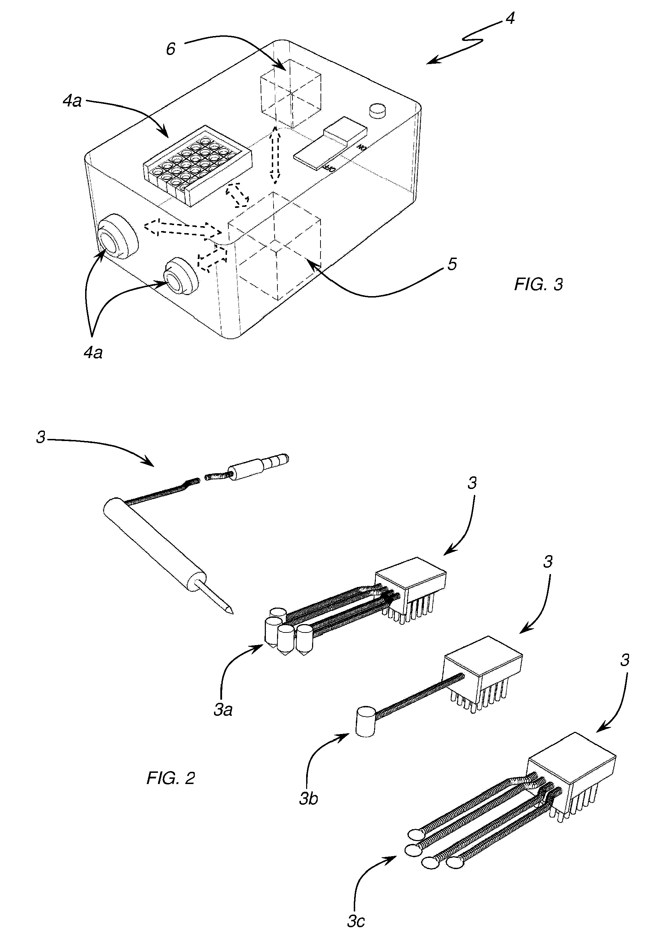

Typically, the interchangeable terminals **3** may be of the kind emitting light rays or emitting laser beams. In other words, they may include, as represented in Fig. 3, red, green or blue laser **3a,** infrared, RGB LED **3b** or others. This allows the execution of treatments such as chromopuncture or laser puncture.

Moreover, the terminals **3** may be electricity emitters **3c** for the execution of transcutaneous electric nerve stimulation (TENS), electro-acupuncture or others.

Another kind of the terminal **3** is constituted by the terminals for the execution of electrocardiograms or the like.

According to another aspect of the invention, the interchangeable terminals **3** are removably coupled to remote devices **4** operatively connected with the central unit **2.** In fig. 3 it is noted that this coupling is achieved by means of multi-contact connectors **4a,** but this should not be considered as limiting for different embodiments.

Furthermore, each remote device **4** comprises a logical control unit **5** that allow to interface control signals and communication data between the central unit **2** and any one of the interchangeable terminals **3** coupled with remote devices **4** by means of the connectors **4a** to allow the execution of a specific therapeutic treatment or a specific diagnostic test.

In other words, the central unit **2** controls the interchangeable terminals **3** by means of remote devices **4** that act as "signal translators".

It is known that the interchangeable terminals **3** are functionally and physically different each other in accordance with the treatment or test that they must execute. In this sense, both the control signals and the data signals are different for each terminal **3,** the latter relating to electrical optical or acoustic levels detected by the terminals **3** or that they must generate. The remote devices **4,** therefore, convert the signals exchanged with the central unit **2,** which will have a unique protocol independent from the kind of interchangeable terminal **3** to control, with the proper signals for each terminal **3.** This is made easier by the presence of the logical control unit **5** which can be suitably programmed to communicate properly with the central unit **2** and consequently to control any kind of interchangeable terminal **3** that is coupled to the remote device **4.**

Advantageously, therefore, the medical apparatus **1** allows the execution of different kinds of diagnostic tests and therapeutic treatments while presenting a single central unit **2** and a single kind of remote devices **4.** All that changes from one treatment to another or between tests is the kind of interchangeable terminals **3** used and the procedure selected on the central unit **2.**

According to another aspect of the invention, more interchangeable terminals **3** can be simultaneously coupled to each remote device **4.** In particular it is noted that the interchangeable terminals **3** can have more than one simultaneous application points. This is to allow the execution of treatments or tests that require a plurality of application points using a single remote device **4.**

Obviously, this must not be considered as limiting for different embodiments of the invention in which, for example, only one interchangeable terminal can be coupled to each remote device and each interchangeable terminal being characterized by presenting a single contact element with the human body.

The number of remote devices **4** should also not be considered as limiting the invention. The remote devices **4** are universal in respect of the interchangeable terminals **3,** but also this fact should not be considered as limiting for different embodiments of the invention in which, for example, for each kind of terminal there is a specific kind of remote device.

In any case, it is evident the cost saving of the invention compared to the purchase of a number of specific medical devices for each test or treatment. Furthermore, the handling is simplified since only the rearrangement of interchangeable terminals **3** is necessary to perform a different kind of test or treatment.

According to a further aspect of the invention, the remote devices **4** comprise a wireless communication circuit **6** for the connection to the central unit **2.** Advantageously, therefore, the wireless link between the central unit **2** and the remote devices **4** allows to simplify tests treatments. Obviously, the central unit **2** is equipped with an equivalent communication circuit **7.**

As the medical device **1** allows to run simultaneously multiple tests and treatments (just multiplying the number of remote devices **4** and of the corresponding interchangeable terminals **3** in use) the absence of cables simplifies the setup of tests and treatments. The absence of cables should not be considered limiting for different embodiments of the invention according to which they are used to connect the central unit with the remote devices. However, it is evident that the use of wireless links allows to optimize the use of the medical apparatus **1** of the invention.

An hospital or similar organization can then establish a plurality of small outpatients' department or room where to treat the patients using only one central unit **2** arranged in a dedicated room. Therefore it is evident the optimization of the used spaces that allows a further economic saving.

The arrangement of a single patient each room and the absence of the central unit **2** in the same room allows to use particularly small rooms. The arrangement of the interchangeable terminals **3** on the patients will be facilitated by the absence of connection cables between them and the central unit **2.** The only wiring, if present, will be that between the used remote device **4** ( at least one each room), and the interchangeable terminals **3.**

Furthermore, it is noted that the movable connection between interchangeable terminals **3** and remote devices **4** can be made by rigid contacts or through cables. In the first case the assembly terminals **3 -** remote device **4** will be miniaturized to allow the stable arrangement of this assembly on the body of the patient. In the second case, instead, the wiring allows to have the remote device **4** (or remote devices **4** if they are used more than one for each patient) comfortably near the patient.

According to another aspect of the invention, since the central unit **2** can be arranged in a different room in respect of the remote devices **4** with the terminals **3,** the control signals exchanged between the central unit **2** and the remote devices **4** of the medical apparatus **1** comprise a first control signal to indicate the kind of interchangeable terminal **3** in use. This allows the operator which acts on the central unit **2** to control all the terminals **3** arranged on each patient to avoid possible mistakes.

Since also the operational state of the terminal **3** is important, among the control signals exchanged between the central unit **2** and the remote devices **4** there is also a second control signal working in this sense.

It is evident that multiple different control signals can be provided, all aimed to simplify the workability of the medical apparatus **1** of the invention and make safer the entire procedure.

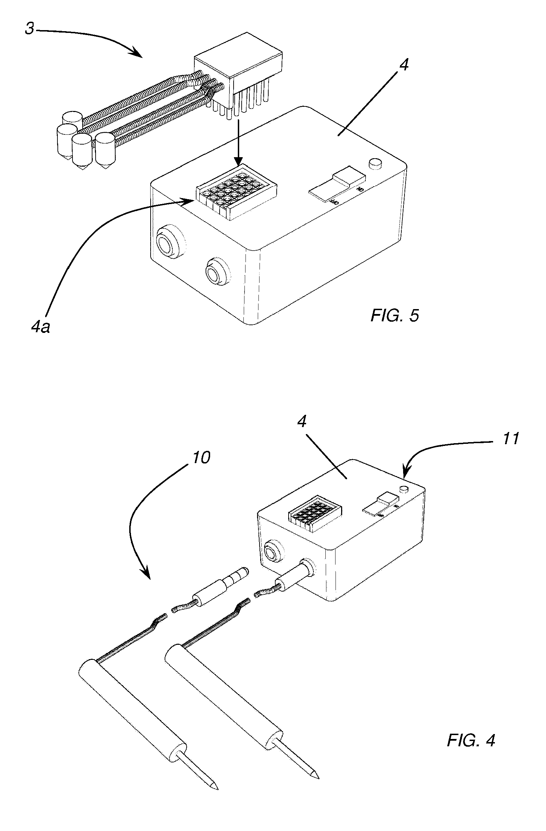

According to another aspect of the invention, the medical apparatus **1** also comprises, as shown in Fig. 4, service terminals 10 removably connectable to each remote device **4** and arrangeable on the patient's body to locate the best position of the interchangeable terminals **3.**

In this sense, a third control signal is sent by the central unit **2** to the remote device **4** to inform it when the service terminal **10** is arranged in such best position. This information can be carried out by means of a LED **11.**

Advantageously, therefore, the arrangement of the interchangeable terminals **3** on the patient's body is not only entrusted to the expertise of the staff, but it is the medical apparatus **1,** by the central unit **2,** which locate the best position by sound or visual signaling when the service terminal **10** touches said position. The staff will know in that moment the best location in which the interchangeable terminal **3** must be arranged.

An example of embodiment for the service terminal **10** is constituted by an ohmmeter, but it is evident that this is only an example of embodiment and that must not be considered as limiting for other embodiments, each suitable to highlight the best location for the Interchangeable terminals **3** during tests or treatments different each other. In the case shown in the figure the ohmmeter is obtained by two leads. However, also this particular should not be considered limitative for different embodiments of the invention where there is a common ground, for example made on the bottom of the remote device, and one or more leads for the operator. Moreover, this embodiment contributes, as known, to locate the best position instead of an area as in the embodiments previously described.

In the described embodiment the control of the best location of the terminals is refered to the central unit **2.** Also in this case it is a non-limiting example for different embodiments where the best location is refered to the logical control unit comprised on the remote devices. Other embodiments may provide a combination of the two previous situations to meet the specific requirements of each test and treatment.

This aspect of the invention allows also a further advantage. Because the application of the interchangeable terminals **3** is made easier, it can be performed even by unskilled person (for example simple attendants) being necessary the presence of skilled persons of the various kind of tests and treatments only on the central unit **2** where they must be checked the terminals **3** used, their workability and their correct arrangement and where, moreover, must be set to the various kind of tests and treatments with the relevant parameters related to not only the kind of test or treatment, but also to the specific patient.

Once again it is clear the economic saving being required the presence of a single skilled person to perform all tests and treatments, all other required people being able to be composed by non-skilled persons.

It is also noted that the embodiment of the central unit **2** is simplified. In fact, as represented in the figures, it can be constituted by any electronic processor provided with a wireless communication circuit **7** and with control software for the remote devices **4.** This software will comprise all routines needed for the selection of different tests or treatments for each remote device **4** connected or for each group of remote devices **4,** and the ability to change the settings for each treatment or test.

In this sense, therefore, it is economic saving for the hospital, the clinic or for any other entity, also the central unit **2.** It may even be constituted by a normal PC by applying the communications circuit **7** and installing the described software.

With regard to remote devices **4,** they are provided with power means typically consisting in, but not necessarily, batteries. The latter may be rechargeable so the remote devices **4** are also provided with a charging electronic circuit and of a cable for the connection to power sources.

Operatively, the patients are each laid in a respective room. The staff then connect, as shown schematically in Fig. 5, to the remote devices **4** in each room the appropriate kind of interchangeable terminals **3** depending on the kind of test or therapeutic treatment which the patient must undergo.

Subsequently, the staff identifies the best location for the terminals **3** on the respective patients also by using service terminals **10.** After this step, the service terminals **10** can be disconnected from the respective remote device **4.**

Meanwhile, on the room where is arranged the central unit **2,** the skilled person verify that the terminals **3** in use on each patient are the correct ones and are all workable. He can also check that their location is correct. All that is made by the exchange of the appropriate control signals between the remote devices **4** and the central unit **2.**

After this step, each patient the skilled person initialize the control software routine dedicated to each specific test or treatment. Next there is the start of each treatment and test.

At the end of each test and treatment, the interchangeable terminals 3 are detached from the patient that can live the room. It is so possible to start with another test or treatment which may be different from the previous one. The staff will then use the same terminals **3** or replace them with another kind suited to the new test or new treatment. Similarly, the skilled person provides to set the central unit **2** to perform, in that specific room and so with those specific remote devices **4,** the medical tests or therapeutic treatments required for the specific patient.

In the light of the foregoing, it is understood that the medical apparatus of the invention overcomes the drawbacks of the known technique allowing to perform simultaneously both a plurality of therapeutic treatments and a plurality of diagnostic tests on different patients.

The same medical apparatus allows, as shown, to reduce overall costs for a clinic or a hospital authority that want to perform said tests and said treatments in terms of space, equipment and staff needed.

The possibility of using any number of remote devices for each central unit and a large number of interchangeable terminals for each remote device allows the use of the single medical apparatus of the invention for the simultaneous execution of a plurality of different tests or different therapies.

The medical apparatus of the invention is susceptible of a number of changes and variants, within the inventive concept disclosed in the appended claims. All the details thereof may be replaced by other technically equivalent parts, and the materials may vary depending on different needs, without departing from the scope of the invention, as defined by the claims.

While the medical apparatus of the invention has been described with particular reference to the accompanying figures, the numerals referred to in the disclosure and claims are only used for the sake of a better intelligibility of the invention and shall not be intended to limit the claimed scope in any manner.

## Claims

1. A medical apparatus, susceptible to be used on a human body to perform one or more therapeutic treatment or to perform one or more diagnostic tests, including:
- at least one programmable central control unit **(2);**
- one or more remote devices **(4)** operatively connected with said central unit **(2);**
- two or more interchangeable terminals **(3)** in combination or alternatively removably connected to said remote devices **(4)** and susceptible to exchange electrical, optical, acoustic and/or magnetic signals with the human body, each of said two or more terminals **(3)** being designed to perform a specific therapeutic treatment or a specific diagnostic test different from the therapeutic treatments or the diagnostic tests executable by the other of said two or more interchangeable terminals **(3),**
each of said remote devices **(4)** comprising at least one logical control unit **(5)** of said interchangeable terminals **(3)** to interface control signals and data communications between said central unit **(2)** and each of said interchangeable terminals **(3)** temporarily connected to said remote device **(4)** to perform specific therapeutic treatments or specific diagnostic tests performable by said interchangeable terminals **(3).**

2. Medical apparatus according to claim 1, wherein said control signals comprise at least one first control signal sent by said remote device **(4)** to said central unit **(2)** to inform it about the kind of said interchangeable terminal **(3)** connected to said remote device **(4).**

3. Medical apparatus according to claim 1 or 2, wherein said control signals comprise at least one second control signal sent by said remote device **(4)** to said central unit **(2)** to inform it about the workability status of said interchangeable terminal **(3)** connected to said remote device **(4).**

4. Medical apparatus according to any of the preceding claims, comprising at least one service terminal **(10)** removably connected to said remote device **(4)** and susceptible to be used on the human body to detect the best location of one or more of said interchangeable terminals **(3).**

5. Medical apparatus according to claim 4, wherein said control signals comprise at least one third control signal sent by said central unit **(2)** to said remote device **(4)** to inform it when the location of said service terminal **(10)** corresponds to the best location of at least one of said interchangeable terminals **(3).**

6. Medical apparatus according to any of the preceding claims, wherein said remote devices **(4)** comprise at least one wireless communication circuit **(6)** for the connection with said central unit **(2).**

7. Medical apparatus according to any of the preceding claims, wherein said interchangeable terminals **(3)** comprise at least one light transmitting terminal.

8. Medical apparatus according to any of the preceding claims, wherein said interchangeable terminals **(3)** comprise at least one laser beam transmitting terminal.

9. Medical apparatus according to any of the preceding claims, wherein said interchangeable terminals **(3)** comprise at least one electricity emitting terminal to perform electrical stimulation.

10. Medical apparatus according to any of the preceding claims, wherein said interchangeable terminals **(3)** comprise a plurality of terminals to perform an electrocardiogram.
